## Europäisches Patentamt

## European Patent Office

## Office européen des brevets

(19)

(11) Publication number: **0 201 211**
A1

(12) **EUROPEAN PATENT APPLICATION**

(21) Application number: **86302608.4**

(22) Date of filing: **09.04.86**

(51) Int. Cl.⁴: **G 01 N 33/543**, G 01 N 33/545, G 01 N 33/546, G 01 N 33/533

(30) Priority: **10.04.85 US 721574**

(43) Date of publication of application: **12.11.86**
**Bulletin 86/46**

(84) Designated Contracting States: **DE FR GB IT**

(71) Applicant: **WHITTAKER CORPORATION, 10880 Wilshire Boulevard, Los Angeles, California 90024 (US)**

(72) Inventor: **Khalil, H. Mohammed, 113 Valleyview Way, SO, San Francisco California 94080 (US)**
Inventor: **Pourfarzaneh, M. T., 1108 Lynn Way, Sunnyvale California 94087 (US)**

(74) Representative: **Day, Jeremy John et al, REDDIE & GROSE 16 Theobalds Road, London, WC1X 8PL (GB)**

(54) Method and compositions for visual solid phase immunoassays based on luminescent microspheric particles.

(57) An immunoassay reactant and technique to detect the presence of analytes visually are disclosed. The technique involves incorporating a luminescent marker substance into microspheric particles which are labelled with a substance having an affinity for the analyte or a complex containing analyte. The results of the assay may be visualised with a simple fluorometer or a light box.

EP 0 201 211 A1

## METHOD AND COMPOSITIONS FOR
## VISUAL SOLID PHASE IMMUNOASSAYS
## BASED ON LUMINESCENT MICROSPHERIC PARTICLES

TECHNICAL FIELD

This invention relates generally to assay methods and compositions for the detection of antibodies, haptens, antigens and other substances of interest in fluid samples. More specifically, the invention relates to visual immunoassay methods and compositions utilizing luminescent microspheres.

BACKGROUND OF THE INVENTION

Many assay procedures have been used to qualitatively and quantitatively determine the occurrence of a wide variety of analytes in biological fluids and other samples. These assay procedures typically exploit a relatively specific affinity between the analyte of interest and a reactant (e.g., enzyme-substrate, antibody-antigen, etc.), at least one of which is labeled with a reporter substance (i.e., one whose presence, either alone or in complex with another substance, is easily detected). Following mixture of the reactant with the sample and incubation, specific analyte-reactant interactions occur forming analyte-reactant complexes. The reaction mixture is subsequently analyzed to detect free and specifically-bound reactant, enabling detection and measurement of the analyte in the sample. Such facile detection is often accomplished with the use of a reporter capable of emitting some type of a signal such as a chromophore, an enzyme, a radioisotope, a luminescent substance, or an optically active substance. Typical analytes include microorganisms, metabolites, proteins, drugs, vitamins, hormones and other substances. These

0201211

analytes usually occur in minute concentrations and their detection requires assay procedures with corresponding sensitivities.

Assay procedures can be divided into two general categories, homogeneous and heterogeneous. In a homogeneous assay, the signal emitted by the specifically-bound-labeled reactant is different from the signal emitted by the free labeled reactant. Hence, the bound and free forms can be distinguished without physical separation.

The archetypal homogeneous assay is the enzyme-multiplied immunoassay technique (EMIT). The substrate-labeled fluorescent immunoassay is another homogenous assay (Burd, J.F. et al., (1977) Clin. Chem. 23:1402; Burd, J.F. et al., (1977) Anal. Biochem. 77:56; and Kohen, F. et al., (1979) J. Steroid Biochem., 11:161).

Homogeneous assays have the advantage of being rapid, easy to perform, and readily amenable to automation. Their principal disadvantages are that they are relatively prone to interferences, are generally applicable only to low molecular weight analytes, and are limited in sensitivity to approximately $10^{-9}$ M.

These disadvantages are significant in a clinical setting where the analyte of interest is a high molecular weight macromolecule or pathogenic microorganism. Further, many analytes of interest are present in biological fluids in very minute concentrations and may go undetected by such homogeneous assay techniques.

In a heterogeneous assay, the signal emitted by the specifically-bound labeled reactant is indistinguishable from the signal emitted by the free labeled reactant. Therefore, a separation step is required to distinguish the two, involving expenditures of time and labor. However, they are in general more sensitive than homogeneous assays and less prone to interference, since interfering substances can be removed in the washing steps. Typical heterogeneous assays include the radioimmunoassay (RIA), the enzyme-linked immunosorbent assay (ELISA) and fluoro-immunoasays (FIA).

Of these assays, RIA and ELISA, although sufficiently sensitive to detect small amounts of analyte, suffer from several drawbacks. For example, RIA presents radiation hazards to the operator, requires special licensing from regulatory agencies and utilizes reagents which have relatively short shelf life. ELISA requires a determination of enzymatic activity which prolongs the assay and adds to its costs and complexity. In addition, RIA and ELISA require highly trained and skilled technicians to perform the assay and relatively expensive instruments to evaluate the results of the assay.

The luminescent immunoassays have the capability to address most of the drawbacks described above but are, in general, described to be less sensitive than RIA and ELISA. However, while this may be true for the serum-based homogenous assays, where the Rayleigh and Raman scattering caused by the high protein content in the solution and the intrinsic fluorescence from certain amino acids residue reduces the signal-to-background ratio, it is not necessarily true for the heterogenous assays. One promising heterogenous technique involves the use of a solid support (e.g., paper disk, plastic dip stick, walls of the test tube and glass or plastic beads) to which an antibody or antigen is absorbed or covalently bonded. This immunoreactive solid surface is then exposed to serum and labeled ligand. The latter react immunologically with the antigen-antibody complex on the solid surface.

After a brief wash to remove unreacted serum and unbound labeled ligand, the signal from the antigen-antibody complex on the solid phase is measured by, e.g., a fluorometer or a simple light box.

There has been continuing interest in further improving the sensitivity of heterogenous luminescent immunoassays. Furthermore, there is a need for developing simpler and more efficient heterogenous luminescent immunoassays which will not require a highly skilled technician to perform. Such assays should measure a wide variety of analytes and provide quantitative or qualitative results employing only simple, inexpensive instrumentation or by simple visual inspection. These improvements will ensure a more rapid and widespread adaptation of such luminescent immunoassays in cost-conscious laboratories.

It is thus an object of the present invention to provide an improved heterogenous luminescent immunoassay which is capable of quantitatively or qualitatively detecting the presence of low concentrations of an analyte in a sample.

It is another object of the present invention to provide a method to visually detect the presence of an analyte in a sample.

It is a further object of the present invention to provide an assay reactant capable of intensifying the signal produced in heterogenous luminescent immunoassays, such that even minute amounts of an analyte may be visually detected.

BRIEF DESCRIPTION OF THE DRAWINGS

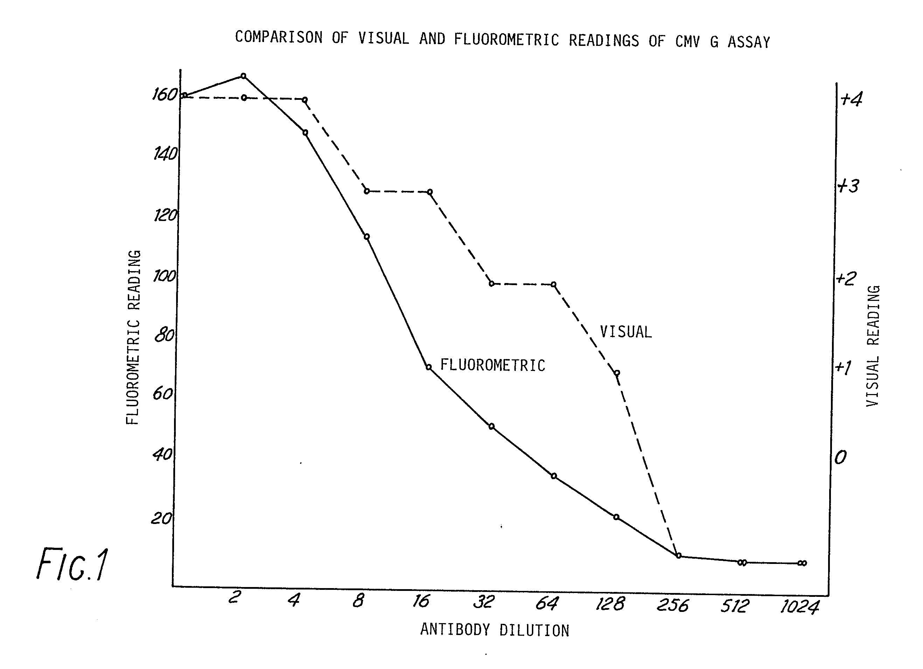

Figure 1 is a graph comparing visual and fluorometric readings of the results of an assay for Cytomegalovirus utilizing a method of the present invention.

0201211

## SUMMARY OF THE INVENTION

Methods are disclosed for detecting the presence of sub-nanomolar quantities of an analyte of interest. in a sample wherein the sample is exposed to a reactant having an affinity for the analyte, or a complex containing the analyte, to bind with the reactant. The reactant of the present invention comprises microspheric particles labeled with a reporter substance capable of being detected either alone, in complex, or both, and said microspheres further labeled with a substance also having an affinity for the analyte or a complex containing the analyte. The detection of the presence of the analyte is made by visually determining the association with the analyte-reactant complexes.

## BEST MODE FOR CARRYING OUT THE INVENTION

In accordance with the present invention, samples may be simply and inexpensively assayed for the presence of analytes. The samples may be solutions directly or indirectly derived from virtually any source including biological sources such as cell cultures, tissues, feces, saliva, urine, blood and other fluids.

The method depends upon incorporating a substance whose presence is detectable by the emission of signals (hereinafter "reporter") in large numbers into polymeric microspheric particles to intensify the signals emitted. The reporter substance is distributed throughout the microspheric matrix and may be composed of any luminescent substance, preferably a fluorophore. These microspheres can themselves be labeled with a reactant having an affinity for the analyte of interest or a complex thereof and thereby signal the presence of the analyte by associating with it. Where the reporter is a luminescent substance, a simple fluorometer or a light box with simplified optics may be used to visually detect the presence of the analyte.

The microspheres may conveniently be spherical in shape and could vary in size from 0.1 to 10 microns. These could be made from any suitable water insoluble polymeric material which, desirably reduces non-specific binding with substances expected to be present in a sample such as polystyrene, a latex polymer. Other latex polymers, both homopolymers (produced by the copolymerization of a single type of monomer, e.g., polystyrene and polyvinyltoulene) and copolymers (produced by the polymerization of more than one type of monomer, e.g., styrene-divinylbenzene, styrenebutadiene, vinyltoluene-t-butylstyrene, and styrene-vinylcarboxylic acid) may be useful in the construction of polymeric microspheres.

The polymerization process itself is a complex chemical phenomenon, the mechanism of which has been the subject of several studies. Essentially, the polymerization process may be made to occur in an emulsion process with an emulsifier producing latex particles smaller than 3 microns, through a seeded emulsion process producing particles 3 microns or more in diameter, or through an emulsifier-free process.

The emulsion polymerization process requires a monomer, water, an emulsifying (surface-active) agent such as potassium laurate, sodium dodecyl sulfate, or sodium dihexysulfosuccinate, and a free-radical-initiating agent such as potassium persulfate or hydrogen peroxide. The polymerization process involves a particle-nuclear initiation phase and a subsequent particle growth phase. In preparing for the reaction, the monomer is first emulsified in water with the surface-active agent, forming soap micelles. The free-radical initiating agent is then added and the emulsion is heated, decomposing the initiator and releasing radicals which, according to some investigators, begin forming particle nuclei in the soap micelles of the suspension. The reaction process involves the initiation (particle-nuclear) phase in which the radicals react with the monomer to create oligomeric radicals, which then further combine with excess monomer to create the polymer. The resulting suspended latex particles have on their surface fixed functional groups, such as amino, carboxyl, hydroxyl, sulfate or amido groups, depending upon the monomer used in the polymerization process. Other functional groups, derived mainly from the emulsifying agent, may be loosely adsorbed to the latex particle surface. These nonfixed groups are removed by mixed-bed ion exchange, dialysis or other separation methods.

In the seeding emulsion polymerization process, which is used to obtain latex particles of 3 microns or greater, the monomer, added to an existing suspension of latex particles, enters these particles, causing them to swell in size. In the emulsifier-free process, a highly concentrated persulfate initiating reagent, a phosphate or carbonate buffer solution, and polymerization at a near-neutral pH are used to produce a polymeric latex that requires no emulsifier-removal step, and which can therefore be used directly.

The number of particles formed in the emulsion of polymerization process depends upon the concentration of the emulsifying agent and the availability of the free radicals that are generated. The size of the particles depends upon the availability of the monomer, and is inversely proportional to the concentration of the free-radical-initiating reagent.

A representative, but by no means exhaustive, list of suitable luminescent substances is set forth in U.S. Patent No. 4,256,836 to Zuk et al. Preferably, the luminescent substance should have primary functionalities as those of xanthene, a fluorophore. However, they may fall into a variety of other groups having other primary functionalities such as: 1- and 2-aminonaphthalene; p,p'-diaminostilbenes; pyrenes; quaternary phenanthridine salts; 9-aminoacridines; p,p'-diaminobenzophenone imines; anthracenes; oxacarbocyanine; merocyanine; 3-aminoequilenin; perylene; bis-benzoxazole; bis-p-oxazolyl benzene; 1,2-benzophenazin; retinol; bis-3-aminopyridinium salts; hellerbrigenin; tetracycline; sterophenol; benzimidazolylphenylamine; 2-oxo-3-chromen; indole; 7-hydroxycoumarin; 4,5-benzimidazoles; 2,3-dihydro-1,4-napthalazinedione; 2,4,5-triphenylimidazole; phenoxazine; salicylate; strophanthidin; porphyrins; triarylmethanes; flavin; and rare earth chelates etc.

The microspheres will also be labeled with a substance having an affinity for the analyte or a complex containing the analyte, such as antigen or antibody. The selected substance is immobilized on the surface of the microsphere by simple absorption or by complex coupling via one of a variety of functional groups on the surface of the microspheres. Use of similar microspheric particles is disclosed in U.S. Patent Nos. 3,853,987, 4,108,972 and 4,224,198 and K. E. Hechemy et al., "Latex Particle Assays in Laboratory Medicine. Part I.," Laboratory Management (June, 1984), 27-40.

A variety of protocols can be used to perform the present invention. In general, the method of the present invention comprises bringing together in a medium (1) a solid-phase-immobilized substance having affinity for said analyte, (2) the sample containing the analyte, and (3) a reagent comprising luminescent labeled microspheres coated with a substance having an affinity for the analyte or a complex containing the analyte.

Thus, an embodiment of the present invention utilizes a one step competitive binding assay where solid-phase-immobilized analyte is brought in contact with a mixture of sample suspected of containing the analyte, and the assay reagent. After a brief incubation, the unbound reagent is washed off and the reagent bound to the solid surface is detected.

In another embodiment of the present invention involving a heterogeneous assay procedure, the presence of an analyte may be detected by sandwich-type assay. The substance having an affinity for the analyte of interest is applied onto a solid support (substrate) to facilitate separation of the complexes to be assayed from the remaining unreacted materials. The coated substrate is then exposed to a sample suspected of containing the analyte of interest under conditions which allow the analyte to associate and form complexes with the reactant fixed onto the solid phase. The associated and unassociated analyte fractions are then separated and the signal-emitting microspheres are applied to the analyte-reactant complexes. The presence of the analyte is determined by detecting the presence of signal-emitting microspheres on the substrate.

Yet another embodiment of the present invention employs the avidin-biotin interaction. In this embodiment, biotinylated anti-human protein is exposed to the analyte-reactant complex under conditions which allow the biotinylated anti-human protein to associate with the analyte-reactant complex. The biotinylated anti-human protein associated with the analyte-reactant complex is then separated from the unreacted biotinylated anti-human protein. The signal-containing microsphere is labeled with avidin and applied to the biotinylated association. The presence of the analyte of interest is determined by detecting the presence of the biotinylated association on the substrate as indicated by the presence of signal-emitting microspheres on the substrate.

The method of the present invention may be used to visually detect the presence of a variety of analytes or analyte-complexes, even those that are typically found in serum in low concentrations such as haptens, antibodies and antigens. A representative, but by no means exhaustive, list of such analytes and antibodies against and antigens derived from various substances and organisms is set forth in U.S. Patent No. 4,256,834 to Zuk et al. and complexes thereof. The method is particularly suited for the visual detection of the presence of antibodies against and/or antigens derived from Herpes, Toxoplasma, Varcella zoster, Cytomegalovirus, Epstein-Barr, Treponema, Rubella, Mycoplasma, Brucella, Gonorrhea, Hepatitis, Rotorvirus, Chlamydia, Respiratory Syncytial Virus, Streptococcus, Neisseria meningitidis, Neisseria gonorrhea, Influenza A and B, Parainfluenza, Salmonella, Hemophilus influenzae, Human Tumor Leukemia Virus, Canine Heart Worm, Canine Pavro Virus, Feline Leukemia Virus, Feline Infectious Peritonitis, Bovine Brucellosis, Avian Infectious Bronchitis, Swine Psuedo Rabies, New Castle Disease Virus, Swine Rotovirus, interferon, amphetamines, cannabinoids, methaqualone, barbituates, cocaine metabolites, opiates, benzodiazepine, methadone and phenylcyclidine.

The method of the present invention may also be used in homogeneous assay procedures where unreacted microspheres may be distinguished from those complexed with the analyte of interest. In such procedures, a substrate would not be necessary.

0201211

EXPERIMENTAL

### Preparation of Anti-Human IgG Coated Fluorescent Microspheres

A commercially available fluorescent latex bead preparation, (SW-07, Carolina Chemical Company, Charlotte, N. Carolina) was used in the experiments described below. The beads were approximately 4 microns, had excitation maxima at 536 and emission maxima at 587 and were coated with goat anti-human IgG or IgM using the standard glutaraldehyde technique.

### Light Box

The light box, for visual readings, was made from 0.125 thick aluminum metal (dimensions: 14" long, 12" high and 7.5" deep) which was painted black. An opening into the light box was covered with black rubber to facilitate entry into the box, while excluding extraneous light. The box was fitted with a tungsten lamp (General Electric, BDK, 100W) which was covered with a metal film, band pass interference filter to reduce the background light. According to the manufacturer's specifications, this filter transmits approximately 60% of the total emittance of light source at 540 nm (10 nm band width). To further reduce the background light, the viewing window was covered with a transparent cellulose acetate color filter (0.01" thick) which transmitted no light at 540 nm and 50% of the total emittance of light at 590 nm. The critical wavelength range, of course, will depend upon the reagents used.

## Preparation of Antigens

The antigens used in the examples described hereinbelow were prepared in the following manner:

### 1. Rubella Antigen

The rubella antigen was prepared by growing Vero D cells in roller bottles containing Eagle's minimum essential medium and 5% newborn calf serum for eight days. The confluent monolayers were infected with rubella virus strain HPV-77 and 48 hours after the infection the bottles were harvested daily for five days. The cell debris was removed by low speed centrifugation. The clarified viral antigen was concentrated, centrifuged at low speed to remove any residual debris and loaded onto a sucrose gradient (35% and 45% W/W). The gradient was centrifuged at 25,000 RPM for 16-20 hours at 8°C in the Beckman L8-55 ultracentrifuge. The viral bands were collected and checked for purity by polyacrylamide gel electrophoresis.

### 2. Cytomegalovirus Antigen

The antigen for the Cytomegalovirus assay was purchased from Viral Antigen Inc. of Tennessee. According to the manufacturer's specifications the antigen was produced by infecting cells with Cytomegalovirus strain AD 169. The infected cells were then extracted with glycine buffer to recover the antigen.

### 3. Herpes simplex Antigen

The antigen for the Herpes simplex virus assay was also purchased from Viral Antigen Inc. of Tennessee. According to the manufacturer's specification, the antigen was produced by infecting Vero cells with Herpes simplex virus McIntyre strain. The infected cells were sonicated and extracted with glycine buffer to recover the antigen.

4.    Toxoplasma Antigen

The antigen substrate for the Toxoplasma assay was purchased from Microbiological Research Corporation and consisted of Toxoplasma gondii strain RH, harvested from peritonial fluid of infected mice.  To obtain the soluble antigen, the suspension was diluted 1:20 with PBS and sonicated at 4°C for nine minutes.  The homogenate was centrifuged at 10,000 rpm for 30 minutes at 4°C to remove insoluble particulate material.  The supernatant was separated from the pellet by careful pipeting.

5.    Treponema pallidum Antigen

The Treponema pallidum antigen was supplied by David Cox of Texas College of Ostopathic Medicine, Forth Worth, Texas.  According to the supplier's specification, the antigen was propagated in a cell culture comprised of cottontail rabbit epithelium cells (Sf1Ep).  The Treponema pallidum cells were separated from the cell cultures by centrifugation and sonicated in PBS prior to adsorbing on the solid phase.

6.    Alpha-Interferon Antigen

The alpha-interferon was supplied by Dr. Karl Thomae GmbH of West Germany.  According to the supplier's specifications, interferon was induced in serum-free medium with sendai virus (strain cantell) in butyrate-treated cells.  The two cell lines used (NAMALWA Clone 8 and NC-37) were both derived from Burkitts lymphomas.  Both lines were propagated in RPMI 1640 and 10 percent fetal calf serum.  IFN was concentrated by precipitation with 65 percent saturated ammonium sulfate.  Before the assay, the pellet was dissolved in pH 0.025M bis-Tris buffered to pH 7.1 and dialyzed to remove residual ammonium sulfate.

Except for alpha-interferon antigen, the above-described antigens were diluted in PBS buffer (Rubella 1:80, Herpes simplex virus 1:20, Cytomegalovirus 1:50, Toxoplasma 1:10, and Treponema pallidum 1:6). An aliquot of the diluted antigen (40 microliters for StiQ Sampler and 200 microliters for microtiter wells) was· spotted on the solid phase and incubated overnight at 4-8°C in a moist chamber. The antigen solution was discarded and the solid phase was incubated for one hour in 0.01M Tris buffer containing 1% BSA and then dried at room temperature and stored at -20°C.

### Example 1

This example illustrates the comparison of visual and fluorometric readings of the titration of a high titered Cytomegalovirus serum sample. Polystyrene microtiter wells were used as the solid phase.

The antigen coated microtiter wells were incubated with 10 microliters of two-fold dilutions of serum in 300 microliters of Tris-Tween® 80-1% BSA buffer (dilution buffer). After 60 minutes, the serum dilutions were discarded and the wells were washed with Tris-Tween® 80 buffer (wash buffer). The wells were then incubated with 300 microliters of a suspension of microspheres in the incubation buffer. After 30 minutes, the microsphere suspension was discarded and the wells were washed with the wash buffer.

The results were evaluated visually in the light box and fluorometrically by a Perkin-Elmer fluorometer. The wells showing visible fluorescence in the light box were deemed positive and the wells showing no fluorescence were deemed negative. Figure 1 illustrates the comparison of the visual and the fluorometric readings of the assay. The two readings gave similar end points at a dilution of 1:256. This correspondence demonstrates that the assay results using microspheres can be read visually using an inexpensive light box with little sacrifice in the sensitivity, since the visual end point closely approximates the fluorometric end point.

## Example 2

This example illustrates the comparison of visual assay for the detection of IgG antibodies to Cytomegalovirus, with various methods currently used for the detection of Cytomegalovirus antibodies. These include complement fixation (CF), hemaglutination (IHA) and Immunofluorescence (IFA).

A 10 microliter aliquot of serum sample was diluted in 500 microliters of dilution buffer in each test tube. The antigen coated StiQ Samplers were immersed into the diluted serum and placed on FIAX® (IDT, San Jose, CA) shaker for 30 minutes. After a brief buffer wash, the samplers were transferred to test tubes containing a suspension of IgG-labeled microspheres in dilution buffer, and incubated for 30 minutes. After another brief buffer wash, the assay results were evaluated visually in the light box and fluorometrically in the FIAX® fluorometer. The results summarized in Tables I and II below show that the visual assays demonstrate excellent sensitivity and specificity when compared with the reference methods.

### Table I

|        | Titer | | Results with Microspheres | |
|        | CF | IHA | Visual | Fluorometric (Pregain) |
| Sample |    |     |        |          |
|--------|-----|-----|--------|----------|
| 1      | <8  | <8  | N      | 01       |
| 2      | 256 | 8192 | P     | 18       |
| 3      | 8   | 256 | P      | 17       |
| 4      | 16  | 1024. | P    | 10       |
| 5      | 16  | 1024 | P     | 13       |
| 6      | 16  | 512 | P      | 10       |
| 7      | 32  | 2048 | P     | 09       |

| | | | |
|---|---|---|---|
| 8 | 32 | 2048 | P | 09 |
| 9 | 256 | 16 | N/P | 03 |
| 10 | 256 | 256 | P | 08 |
| 11 | 128 | 4096 | P | 18 |
| 12 | 64 | 4096 | P | 19 |
| 13 | <8 | <8 | N | 01 |
| 14 | <8 | <8 | N | 02 |
| 15 | <8 | <8 | N | 01 |
| 16 | <8 | <8 | N | 02 |
| 17 | <8 | <8 | N | 01 |
| 18 | <8 | <8 | N | 01 |
| 19 | 32 | 2048 | P | 15 |
| 20 | 32 | 2048 | P | 15 |
| 21 | 32 | 4096 | P | 14 |
| 22 | 64 | 4096 | P | 14 |
| 23 | <8 | <8 | N | 01 |
| 24 | 32 | 4096 | P | 13 |
| 25 | 16 | 2048 | P | 20 |
| 26 | 8 | 2048 | P | 13 |
| 27 | 8 | 1024 | P | 11 |
| 28 | <8 | <8 | N | 01 |
| 29 | 8 | 256 | P | 12 |
| 30 | 8 | 256 | P | 09 |
| 31 | <8 | <8 | N | 02 |
| 32 | <8 | <8 | N | 01 |
| 33 | <8 | <8 | N | 01 |
| 34 | <8 | <8 | N | 01 |
| 35 | 8 | 1024 | P | 13 |
| 36 | 8 | 1024 | P | 10 |
| 37 | 16 | 1024 | P | 20 |
| 38 | 16 | 1024 | P | 20 |
| 39 | 64 | 512 | P | 09 |
| 40 | 64 | 512 | P | 09 |
| 41 | <8 | <8 | N | 01 |
| 42 | 64 | 4096 | P | 23 |
| 43 | <8 | <8 | N | 01 |

0201211

| | | | |
|---|---|---|---|
| 44 | <8 | <8 | N | 01 |
| 45 | <8 | <8 | N | 01 |
| 46 | <8 | <8 | N | 01 |
| 47 | <8 | <8 | N | 02 |
| 48 | <8 | <8 | N | 02 |
| 49 | 16 | 64 | P | 15 |
| 50 | 64 | 512 | P | 21 |
| 51 | 32 | 512 | P | 17 |
| 52 | 64 | 2048 | P | 18 |
| 53 | 64 | 1024 | P | 24 |
| 54 | 256 | 128 | P | 15 |
| 55 | 16 | 128 | P | 09 |
| 56 | 16 | 128 | P | 08 |
| 57 | 8 | 16 | P | 06 |
| 58 | 16 | 64 | P | 13 |
| 59 | 128 | 64 | P | 08 |
| 60 | 32 | 256 | P | 15 |
| 61 | 32 | 256 | P | 15 |
| 62 | 128 | 512 | P | 19 |
| 63 | 128 | 512 | P | 22 |
| 64 | 64 | 512 | P | 15 |
| 65 | 64 | 1024 | P | 20 |
| 66 | <8 | <8 | N | 01 |
| 67 | 16 | 256 | P | 17 |
| 68 | 16 | 512 | P | 16 |
| 69 | 8 | 128 | P | 15 |
| 70 | 8 | 256 | P | 12 |
| 71 | <8 | <8 | N | 01 |
| 72 | <8 | <8 | N | 02 |
| 73 | 32 | 512 | P | 13 |
| 74 | <8 | <8 | N | 01 |
| 75 | 32 | 1024 | P | 14 |
| 76 | 32 | 1024 | P | 13 |
| 77 | <8 | <8 | N | 01 |
| 78 | 64 | 256 | P | 16 |
| 79 | 64 | 256 | P | 14 |

| 80 | <8 | <8 | N | 01 |
| 81 | <8 | <8 | N | 01 |
| 82 | <8 | <8 | N | 01 |

## Table II

| | | | Results with Microspheres | |
| Sample | IFA Titer | | Visual | Fluorometric (Pregain) |
|---|---|---|---|---|
| 1 | 128 | | P | 13 |
| 2 | 8192 | | P | 13 |
| 3 | 64 | | P | 06 |
| 4 | 512 | | P | 18 |
| 5 | 256 | | P | 10 |
| 6 | 128 | | P | 05 |
| 7 | 128 | | P | 10 |
| 8 | 4096 | | P | 25 |
| 9 | 128 | | P | 10 |
| 10 | 1048 | | P | 16 |
| 11 | <16 | | N | 01 |
| 12 | <16 | | N | 01 |

## Example 3

This example illustrates the detection of IgG antibodies to human Cytomegalovirus in undiluted or diluted human serum employing avidin-biotin interaction. Polystyrene microtiter wells were used as the solid phase.

The Cytomegalovirus antigen-coated microtiter wells were incubated with 300 microliters of undiluted serum or 10 microliters of serum sample diuted in 300 microliters of the dilution buffer. After 60 minutes, the sample was discarded, the wells were washed with the wash buffer, and incubated with biotinylated goat anti-human IgG (purchased from Vector, Burlingame, California) diluted in 300 microliters of the dilution buffer. After 30 minutes the diluted IgG was discarded and wells were washed with wash buffer and incubated with dilution buffer containing avidinized fluorescent microspheres (the microspheres were avidinized using a procedures similar to one described for the preparation of IgG coated microspheres). After 15 minutes, the microsphere suspension was discarded and the wells were washed with wash buffer.

The results of the assay were evaluated in the light box. A total of 11 serum samples were assayed. These samples were previously tested for the presence or the absence of IgG antibodies to Cytomegalovirus by the FIAX® Cytomegalovirus assay. The results summarized in Table III below show that the assay, utilizing diluted or undiluted serum samples, correlates well with the FIAX® Cytomegalovirus assay.

## Table III

| Sample No. | Cytomegalovirus Antibody Status by FIAX® | Results with Microspheres | |
| --- | --- | --- | --- |
| | | Diluted Serum | Undiluted Serum |
| 1 | N | N | N |
| 2 | N | N | N |
| 3 | N | N | N |
| 4 | P | P | P |
| 5 | P | P | P |
| 6 | P | P | P |
| 7 | P | P | P |
| 8 | P | P | P |
| 9 | N | N | N |
| 10 | N | N | N |
| 11 | N | N | N |

## Example 4

This example illustrates the detection of IgG antibodies to Herpes Simplex Virus.

Using an assay protocol similar to one described in Example 2 and employing Herpes Simplex Virus antigen as the substance having affinity for the analyte, several serum samples were assayed. The results summarized below in Table IV show that the sensitivity and the specificity of the visual assay compares well with that of the FIAX® Herpes Simplex antibody assay.

### Table IV

| Sample | FIAX® Titer | Results With Microspheres | |
| --- | --- | --- | --- |
| | | Visual | Flurometric (Pregain) |
| 1 | 400 | P | 28 |
| 2 | 25 | P | 10 |
| 3 | <8 | N | 02 |
| 4 | 30 | P | 10 |
| 5 | 50 | P | 10 |
| 6 | <8 | N | 02 |
| 7 | 90 | P | 16 |
| 8 | <8 | N | 02 |

## Example 5

This example illustrates the detection of IgG antibodies to Toxoplasmosis. The assay was performed substantially as described in Example 4, substituting Toxoplasmosis antigen for the Herpes Simplex antigen. The results summarized below in Table V once again show that the sensitivity and the specificity of the visual assay compares well with that of the FIAX® Herpes simplex virus assay.

Table V

| | Sample | FIAX® Titers | Results With Microspheres | |
| | | | Visual | Fluorometric (Pregain) |
|---|---|---|---|---|
| | 1 | 400 | P | 23 |
| | 2 | 40 | P | 9 |
| | 3 | <16 | N | 01 |
| | 4 | <16 | N | 01 |
| | 5 | 167 | P | 19 |
| | 6 | 74 | P | 05 |
| | 7 | <16 | N | 01 |
| | 8 | <16 | N | 01 |
| | 9 | 62 | P | 04 |

Example 6

This example illustrates the detection of IgM antibodies to rubella virus.

Approximately 10 microliters of serum sample was pipetted into each test tube and preheated for 30 minutes, with 120 microliters of IgG antibodies removing reagent (H. Schmitz et al., Journal of Clinical Microbiology (1975) 1:132-135) and diluted in 320 microliters of dilution buffer. The remaining assay protocol was similar to one described in Example 2 for the detection of IgG antibodies.

A total of 11 serum samples were assayed. These samples were well characterized by the Rubazyme M (Abbott) assay and contained high as well as low levels of IgM antibodies to Rubella virus. The results, summarized in Table VI, show that the assay demonstrates good sensitivity as well as specificity when compared to the Rubazyme assay.

0201211

## Table VI

| Sample | Rheumatoid Factor (IU/ml) | Rubazyme M Index | Results with Microspheres | |
|---|---|---|---|---|
| | | | Visual | Fluorometric (Pregain) |
| 1 | --- | 3 | P | 50 |
| 2 | --- | 2.6 | P | 10 |
| 3 | 2.2 | <0.9 | N | 03 |
| 4 | 3.3 | 3 | P | 44 |
| 5 | 18 | 2.9 | P | 12 |
| 6 | 24 | 2.6 | P | 12 |
| 7 | --- | <0.9 | N | 03 |
| 8 | 800 | <0.90 | N | 02 |
| 9 | 49 | <0.90 | N | 03 |
| 10 | --- | <0.90 | N | 04 |
| 11 | --- | <0.90 | N | 03 |
| 12 | 2.3 | 2.8 | P | 18 |
| 13 | 3.0 | 1.2 | P | 14 |
| 14 | 4.5 | 1.7 | P | 11 |

## Example 7

This example illustrates the detection of IgM antibodies to Toxoplasmosis.

Using an assay protocol similar to one described in Example 6, a total of 17 serum samples were assayed for the presence or the absence of IgM antibodies to Toxoplasmosis. The results, summarized below in Table VII, show that the assay demonstrates good sensitivity as well as specificity when compared to IFA slide test (MRC) for Toxoplasmosis.

## Table VII

| Sample | IFA Toxoplasma M | Results With Microspheres | |
|---|---|---|---|
| | | Visual | Fluorometric (Pregain) |
| 1 | 320 | P | 10 |
| 2 | <10 | N | 01 |
| 3 | <10 | N | 02 |
| 4 | 40 | N | 04 |
| 5 | 80 | P | 10 |
| 6 | 80 | P | 11 |
| 7 | 160 | P | 15 |
| 8 | 40 | P | 11 |
| 9 | 80 | P | 11 |
| 10 | 320 | P | 14 |
| 11 | 160 | P | 7 |
| 12 | <10* | N | 03 |
| 13 | 20 | P | 07 |
| 14 | <10** | N | 03 |
| 15 | <10 | N | 02 |
| 16 | <10 | N | 01 |
| 17 | <10 | N | 01 |

\* High Polar Staining Before and After Treatment
\*\* Low RF, No ANA, Polar Staining

## Example 8

This example illustrates the detection of IgG antibodies to Treponema pallidum.

An assay protocol, similar to one outlined in Example 2 was used for the present example. The StiQ Samplers was coated with $5 \times 10^6$ Treponema pallidum per sampler. A total of 45 serum samples were assayed. These serum samples were previously tested for the presence of anti-Treponema pallidum antibodies by the FTA-ABS test. The results, summarized below in Table VIII, show that the sensitivity and specificity of the visual assay compares well with the FTA test.

## Table VIII

| Sample | Antibody Status by FTA | Results with Microspheres (Visual) |
|---|---|---|
| 1 | P | P |
| 2 | P | P |
| 3 | P | P |
| 4 | P | P |
| 5 | P | P |
| 6 | P | P |
| 7 | P | P |
| 8 | P | P |
| 9 | P | P |
| 10 | P | P |
| 11 | P | P |
| 12 | P | P |
| 13 | P | P |
| 14 | P | P |
| 15 | P | P |
| 16 | P | P |
| 17 | P | P |
| 18 | P | P |
| 19 | P | P |
| 20 | P | P |
| 21 | P | P |
| 22 | P | P |
| 23 | P | P |
| 24 | P | P |
| 25 | P | N |
| 26 | P | N |
| 27 | N | N |
| 28 | N | N |
| 29 | N | N |

| 30 | N | N |
|----|---|---|
| 31 | N | N |
| 32 | N | P |
| 33 | N | N |
| 34 | N | N |
| 35 | N | N |
| 36 | N | N |
| 37 | N | N |
| 38 | N | N |
| 39 | N | N |
| 40 | N | N |
| 41 | N | N |
| 42 | N | N |
| 43 | N | N |
| 44 | N | N |
| 45 | N | N |
| 46 | N | N |

## Example 9

This example illustrates the detection of human alpha-interferon in a sandwich type assay using mouse monoclonal antibodies. The solid phase was comprised of polystyrene microtiter wells.

The microtiter wells were coated with mouse monoclonal antibody (2 micrograms/well) directed against human leukocyte interferon (supplied by Thomae of West Germany) using the coating procedure described above. The fluorescent microspheres were coated with a second mouse monocloncal antibody, directed against human interferon (supplied by the same source as above). The antibody coated wells were allowed to incubate with various dilutions of alpha-interferon. The unreacted interferon was discarded and after washing with wash buffer, the wells were incubated with monoclonal antibody labeled microspheres. The unreacted microspheres were discarded and the wells were finally washed with the wash buffer. Appropriate controls were also included to monitor the non-specific binding. The results of the assay were evaluated in . the light box. The wells showing visible fluorescence were deemed positive while those showing no fluorescence were deemed negative. The detection limit was 1.5 nanograms when the assay time was 7 hours (6 hours first incubation and 1 hour second incubation) and 150 picograms when the assay time was 19 hours (18 hours first incubation and one hour second incubation). The results of the assay are summarized in Table IX below.

TABLE IX

| Interferon Conc. | Results With Microspheres | |
| --- | --- | --- |
| | 7 Hour Assay | 19 Hour Assay |
| 150 ng | P | P |
| 15 ng | P | P |
| 1.5 ng | P | P |
| 0.15 ng | N | P |
| 0.015 ng | N | N |
| Control | N | N |

Example 10

This example illustrates the comparison of the detection of viral antibody (Cytomegalovirus) in human serum versus whole human blood.

Human whole blood samples were obtained from American Red Cross Blood Bank. The blood was collected in vacutainer tubes containing 15% EDTA. To obtain serum, half of the whole blood sample was permitted to clot, stored in the refrigerator overnight and centrifuged at 2000 rpm for 10 minutes. The supernatant serum was removed and stored in the refrigerator.

The serum and blood samples were assayed using the procedure outlined in Example 2. The volume of blood sample assayed was twice as compared to serum (20 microliters of blood vs. 10 microliters of serum) to compensate for the volume of red blood cells in whole blood samples compared to plasma. The results of the assay, summarized below in Table X, show that identical correlation of assay results between serum and whole blood samples was obtained.

Table X

| | IgG Antibody Status for Cytomegalovirus | |
| Sample | Serum | Whole Blood |
| --- | --- | --- |
| 1 | P | P |
| 2 | P | P |
| 3 | P. | P |
| 4 | P | P |
| 5 | N | N |
| 6 | P | P |
| 7 | N | N |
| 8 | N | N |
| 9 | N | N |
| 10 | P | P |
| 11 | N | N |
| 12 | P | P |
| 13 | P | P |
| 14 | N | N |
| 15 | P | P |
| 16 | P | P |
| 17 | P | P |
| 18 | P | P |
| 19 | P | P |
| 20 | N | N |

0201211

| | | |
|---|---|---|
| 21 | N | N |
| 22 | P | P |
| 23 | N | N |
| 24 | N | N |
| 25 | P | P |
| 26 | N | N |
| 27 | P | P |
| 28 | P | P |
| 30 | P | P |
| 31 | N | N |
| 32 | P | P |

C L A I M S

1.   A method for detecting the presence of an analyte in a sample which comprises the steps of :

(a)   providing a reagent comprising microspheric particles labelled with a substance having affinity for the analyte or a complex containing said analyte and with sufficient of a luminescent reporter to permit detection of said reagent;

(b)   exposing said microspheres to a sample suspected of containing the analyte or to a sample which has been contacted with a reactant having affinity for said analyte to form an analyte-reactant complex and allowing said analyte or said complex to associate with said microspheres;   and

(c)   detecting the presence of labelled microspheres associated with said analyte or said complex.

2.   A method according to claim 1, wherein an analyte-reactant complex is used, and said complex is a biotinylated complex and said substance having affinity for said complex is aridin.

3.   A method according to claim 1 or claim 2, wherein said reporter substance is a fluorophore, preferably xanthene, or is selected from 1- and 2-aminonaphthalene, p,p'-diamino-stilbenes, pyrenes, quaternary phenanthridine salts, 9-aminoacridines, p,p'-diamino-benzophenone imines, anthracenes, oxacarbocyanine, merocyanine, 3-amino-equilenin, perylene, bis-benzoxazole, bis-p-oxazolyl benzene 1,2-benzophenazin, retinol, bis-3-amino-pyridinium salts, hellerbrigenin, tetracycline, sterophenol, benzimidazolyphenylamine, 2-oxo-3-chromen, indole, 7-hydroxycoumarin, 4,5-benzimidazoles, 2,3-dihydro-1,4-napthalazinedione, 2,4,5-triphenylimidazole, phenoxazine, salicylate, strophanthidin, porphyrins, triarylmethanes, flavin, and rare earth chelates.

0201211

4. A method according to any one of claims 1 to 3, wherein said microspheres comprise a polymer, preferably a latex polymer, especially polystyrene.

5. A method according to any one of claims 1 to 4, wherein said analyte or said analyte-reactant complex is fixed onto a substrate.

6. A method according to any one of claims 1 to 5, wherein said sample is a fluid sample derived from a biological specimen.

7. A method according to any one of claims 1 to 6, wherein formation of a analyte-reactant complex results in a fraction of complexed analyte and a fraction of uncomplexed analyte; and the complexed and uncomplexed fractions are separated prior to contacting said complex with said labelled microspheres and/or prior to forming a biotinylated analyte-reactant complex.

8. A method according to any one of claims 1 to 7, wherein said analyte comprises an antibody, an antigen or a hapten, preferably an antibody selected from antibodies against Herpes, Toxoplasma, Varcella zoster, Cytomegalovirus, Epstein-Barr, Treponema, Rubella, Mycoplasma, Brucella, Gonorrhea, Hepatitis, Rotorvirus, Chlamydia, Respiratory Syncytial Virus, Streptococcus, Neisseria meningitidis, Neisseria gonorrhea, Influenza A and B, Parainfluenza, Salmonella, Hemophilus influenzae, Human Tumor Leukemia Virus, Canine Heart Worm, Canine Pavro Virus, Feline Leukemia Virus, Feline Infectious Peritonitis, Bovine Brucellosis, Avian Infectious Bronchitis, Swine Psuedo Rabies, New Castle Disease Virus, Swine Rotovirus and interferon or an antigen selected from antigens derived from Herpes, Toxoplasma, Varcella zoster, Cytomegalovirus, Epstein-Barr, Treponema, Rubella, Mycoplasma, Breucella, Gonorrhea, Hepatitis, Rotorvirus, Chlamydia, Respiratory Syncytial Virus, Streptococcus, Neisseria meningitidis, Neisseria gonorrhea, Influenza A and B, Parainfluenza, Salmonella, Hemophilus influenzae, Human Tumor Leukemia Virus, Canine Heart Worm, Canine Pavro Virus, Feline Leukemia Virus, Feline Infectious Peritonitis, Bovine Brucellosis, Avian Infectious Bronchitis, Swine Psuedo Rabies, New Castle Disease Virus, Swine Rotovirus, interferon, amphetamines, cannabinoids, methaqualone, barbituates, cocaine metabolites, opiates, benzodiazepine, methadone, and phenylcyclidine.

9.   A method according to any one of claims 1 to 8, wherein the presence of said microspheres associated with said analyte or said analyte-reactant complex is detected visually, preferably using a fluorometer or a light box.

10.   An assay reactant comprising a microspheric particle having a luminescent reporter therein and labelled with a substance having an affinity for an analyte to be assayed or a complex of said analyte.

11.   An assay reactant according to claim 10, wherein said microspheres are labelled with avidin.

COMPARISON OF VISUAL AND FLUOROMETRIC READINGS OF CMV G ASSAY

FIG.1

## DOCUMENTS CONSIDERED TO BE RELEVANT

EP 86302608.4

| Category | Citation of document with indication, where appropriate, of relevant passages | Relevant to claim | CLASSIFICATION OF THE APPLICATION (Int. Cl.4) |
|---|---|---|---|
| D,Y | US - A - 4 256 834 (ZUK et al.)<br><br>* Column 20, lines 22-64; claim 1 *<br><br>-- | 1 | G 01 N 33/543<br>G 01 N 33/545<br>G 01 N 33/546<br>G 01 N 33/533 |
| D,Y | US - A - 3 853 987 (DREYER)<br><br>* Column 8, lines 60-67 - column 9, lines 1-39 *<br><br>-- | 1,3 | |
| D,A | US - A - 4 224 198 (REMBAUM et al.)<br><br>* Column 9, line 1 - column 10, line 17 *<br><br>-- | 1 | |
| A | DE - A1 - 2 618 511 (MILES LABORATORIES)<br><br>* Pages 124-128 *<br><br>---- | 2 | |

TECHNICAL FIELDS SEARCHED (Int. Cl.4)

G 01 N 33/00

The present search report has been drawn up for all claims

| Place of search | Date of completion of the search | Examiner |
|---|---|---|
| VIENNA | 15-07-1986 | SCHNASS |